# EUROPEAN PATENT APPLICATION

(11) **EP 1 277 472 A1**
(43) Date of publication of application: **22.01.2003**
(21) Application number: 01925953.0
(22) Date of filing: 26.04.2001
(51) Int. Cl.: A61K 31/7016, A61K 31/702, A61K 31/737, A61P 35/00, C12Q 1/02, A23L 1/30, C07H 3/04

(54) **REMEDIES FOR CANCER**

(30) Priority: 28.04.2000 JP 2000131375; 16.06.2000 JP 2000182124; 09.03.2001 JP 2001067472
(71) Applicant: Orient Cancer Therapy Co. Ltd, Mitaka-shi, Tokyo 181-0015 (JP)
(72) Inventor: YAGITA, Akikuni, Mitaka-shi, Tokyo 181-0015 (JP)
(74) Representative: Forstmeyer, Dietmar, Dr. rer. nat., Dipl.-Chem.
(86) International application number: JP0103621
(87) International publication number: WO01082935

(57) **Abstract**

There is provided a therapeutic agent for cancer mainly comprising a saccharide having an α1→3 steric structure where an action of NKT cells on NKR-P1 (natural killer receptor P1) which is a natural killer (NK) cell antigen receptor of NKT cells in an activating ability of natural killer T (NKT) is used as an index and being used in a formulation where the activation can be sustained.

## Description

### Technical Field

The present invention relates to a therapeutic agent for cancer paying attention to activation of NKT cells or relates to a supplementary food preparation for health by oral administration which is taken with an expectation of anticancer effect paying attention to activation of NKT cells.

### Background of the Invention

For the selection of substances useful for the prevention or therapy of malignant neoplasms or cancer, their direct effect to cancer cells has been considered to be important. Although immunopotentiator have been noted to be useful for the therapy of cancer, all of the compounds obtained as the immunopotentiators have a weak anticancer effect and, by a sole immunotherapy or even by a joint therapy with chemotherapy, a satisfactory therapeutic effect for cancer has not been achieved.

Dr. Yagita who is the present inventor has previously paid his attention to the usefulness of a substance which induces interleukin 12 (IL-12) *in vivo* as an epoch-making means in a therapy of cancer, found that AHCC which is a processed product of mycelia of *Cortinellus shiitake* has such a function and established a therapeutic method for cancer which may be called a novel immunotherapy for cancer (NITC). There has been a fact that, although IL-12 has an anticancer effect, it results in a side effect when directly administered *in vivo* and IL-12 *per se* has been unable to be used as an anticancer agent. However, a preparation containing AHCC reported by Yagita has achieved a significant therapeutic and life-prolonging effect in the therapy of cancer. Thus, by administration of an effective amount of AHCC whereby IL-12 can be induced *in vivo,* Yagita has achieved an object of therapy of cancer (Japanese Patent Laid-Open No. 10/139,670).

IL-12 has a potentiating action for the production of interferon γ (IFN γ) and has an activating effect and a potentiating action for natural killer (NK) cells, LAK cells (lymphokine activated killer cells) and killer T cells which play a role of cellular immune *in vivo.* IFN γ is cytokine which induces the immune response of organism to a state where T helper 1 cells (Th1) act. The state in which Th1 acts is a state where NKT cells and killer T cells easily achieve the effect or, in other words, a state where interleukin 2 (IL-2) and IL-12 are abundantly produced. Killer T cells and LAK cells have been known as the cells participating in cancer immune. With regard to NK cells, there are also reports that they participate in anticancer action of organism but activity of NK cells does not correlate to clinical anticancer effect and it has been proved by Yagita that the induced production amount of IL-12 is rather in a completely reversed correlation with the activity of NK cells whereby it has been concluded that NK cells do not participate in the anticancer effect in human being.

At present, it has been established by Yagita that a substance having the ability for inducing the production of IL-12 has a possibility of becoming a prominent anticancer substance.

However, in some patients suffering from cancer, production of IL-12 is not well induced even by administration of AHCC whereby the therapeutic effect is not achieved or, even when production of IL-12 is induced, the therapeutic effect is not achieved. In view of the above, there has been a brisk demand for the development of new therapeutic agents for cancer acting by a mechanism which is different from the anticancer effect of AHCC.

In the action mechanism of cancer immune, it has been known that the amount of cytokine produced or induced in organism is an important factor and methods where cytokine which is said to have an anticancer effect is administered, induced or produced to cure the cancer have been attempted and carried out already. However, although the relation between cancer and immune or that between cancer and cytokine has been clarified, curing of and life-prolonging effects for cancer have been noted only in 50% or less of the patients. NKT cells which have been found in recent years as the cells participating in cancer immune (Cui, J., et al.: *Science*, 278, 1623, 1997) has functions such as a potent cytokine producing ability or, particularly, IFN γ producing ability and cell injury, etc. mediated by Fas and perforin. Accordingly, it is expected that, when NKT cells are activated, curing and life-prolonging effects for patients suffering from cancer are further improved.

Taniguchi, et al. have found a specific glycolipid antigen recognized by Vα24Vβ11 which is a specific T cell antigen receptor (TCR) of NKT cells and reported that the said antigen is an α-galactosylceramide. They have further proved that, in cancer-bearing mice to which α-galactosylceramide is administered, NKT cells are activated and, although disappearance of cancer is not observed, metastasis is suppressed.

It has been reported that, in NKT cells, there is an NK cell antigen receptor (NKR-P1; natural killer receptor P1) as another receptor (Special Issue for Fundamentals and Clinics of NKT Cells: *Saishin Igaku,* volume 55, no. 4, pages 818-823, 2000). NKR-P1 is also related to the activation of NKT cells.

### Disclosure of the Invention

The present inventor has carried out repeated investigations on cancer immune cascade in the prevention or therapy of cancer and found that, in a cascade in which activated NKT cells bearing cancer immunity are participated, actions of the two different antigen receptors concerning activation of NKT cells, i.e. NKR-P1 (natural killer receptor P1) and Vα24Vβ11, are entirely different and that saccharides having a β1→3/1→6 steric structure are insufficient for the action of NKR-P1 but saccharides retaining an α1→3 steric structure can be excellent activators for the receptor. As a result thereof, it is now possible to provide a novel and useful therapeutic agent for cancer having an activating ability for NKT cells.

Thus, an embodiment of the present invention is a composition mainly comprising a saccharide having an α1→3 steric structure where an action of NKT cells on NKR-P1 which is a natural killer (NK) cell antigen receptor of NKT cells in an activating ability of natural killer T (NKT) is used as an index and being used in a formulation where the activation can be sustained.

Another embodiment of the present invention is a. composition where a saccharide having an α1→3 steric structure is a main ingredient, characterized in that, the composition selectively acts on NKR-P1 which is a natural killer (NK) cell antigen receptor of NKT cells in an activating ability of natural killer T (NKT) cells and is used in a formulation where the activation can be sustained.

Another embodiment of the present invention is a composition mainly comprising a saccharide having an α1→3 steric structure which selectively acts on NKR-P1 which is a natural killer (NK) cell antigen receptor whereby a large-scale production of interferon γ (IFN γ) is induced and is used in a formulation which induces the ratio of T helper 1 cell to T helper 2 cell (Th1/Th2) in a direction where an immune system on which Th1 mainly acts works.

Another embodiment of the present invention is the composition mentioned in any of the above, wherein CD 3 and CD 161 which are cell surface markers are measured whereby NKR-P1 which is a natural killer (NK) cell antigen receptor of NKT cells is measured and an activating ability of the natural killer T (NKT) cells is determined.

Another embodiment of the present invention is the composition containing a saccharide having an α1→3 steric structure as a main ingredient which is mentioned in any of the above , wherein it is used by the way selected from any of the following formulations:
1) use for a joint therapy with an anticancer chemotherapeutic agent,
2) use for a joint therapy with a radiotherapy,
3) use for a joint therapy with a steroid therapy and
4) use to patients suffering from cancer where activating ability of natural killer T (NKT) cells lowers by the action to NKR-P1.

Another embodiment of the present invention is a supplementary food preparation for health by oral administration containing any of the compositions mentioned above.

Another embodiment of the present invention is a supplementary food preparation for health by oral administration containing any of the compositions mentioned above, wherein CD 3 and CD 161 which are cell surface markers are measured whereby NKR-P1 which is a natural killer (NK) cell antigen receptor is measured and an activating ability of the natural killer T (NKT) cells is tested.

Another embodiment of the present invention is the supplementary food preparation for health by oral administration containing a saccharide having an α1→3 steric structure as a main ingredient which is mentioned in any of the above, wherein it is used by selected from any of the following formulations:
1) use for a joint therapy with an anticancer chemotherapeutic agent,
2) use for a joint therapy with a radiotherapy,
3) use for a joint therapy with a steroid therapy and
4) use to patients suffering from cancer where activating ability of natural killer T (NKT) cells lowers by the action to NKR-P1.

Another embodiment of the present invention is a commercial medium carrying the information concerning any of the compositions mentioned above.

Another embodiment of the present invention is a commercial method utilizing the information concerning any of the compositions mentioned above.

Another embodiment of the present invention is a screening method for therapeutic agent for cancer where a saccharide having an α1→3 steric structure is a main ingredient, characterized in that, the screening is carried out using an action of NKT cells on NKR-P1 which is a natural killer (NK) cell antigen receptor in an activating ability of natural killer T (NKT) as an index.

Another embodiment of the present invention is a screening method for therapeutic agent for cancer where a saccharide having an α1→3 steric structure is a main ingredient, characterized in that, the screening is carried out using an action of NKT cells on NKR-P1 which is a natural killer (NK) cell antigen receptor in an activation ability of natural killer T (NKT) as an index, wherein activation of the said NKT cell is tested by measuring the NKR-P1 by the measurement of CD 3 and CD 161 which are cell surface markers.

Another embodiment of the present invention is a testing means for judging the usefulness of a composition where a saccharide having an α1→3 steric structure is a main ingredient, characterized in that, the test is carried out using an action of NKT cells on NKR-P1 which is a natural killer (NK) cell antigen receptor in an activating ability of natural killer T (NKT) as an index.

Another embodiment of the present invention is a testing means for judging the usefulness of a composition where a saccharide having an α1→3 steric structure is a main ingredient, characterized in that, the test is carried out using an action of NKT cells on NKR-P1 which is a natural killer (NK) cell antigen receptor in an activating ability of natural killer T (NKT) is used as an index, wherein activating ability of the said NKT cell is tested by measuring the NKR-P1 by the measurement of CD 3 and CD 161 which are cell surface markers.

Another embodiment of the present invention is a commercial method where the above-mentioned testing means is used as a supplementary means in liaison with medical organizations.

### Brief Description of the Drawings

Fig. 1 shows variations in interferon γ (IFN γ) for all cases examined.
Fig. 2 shows variations in interferon γ (IFN γ) for the cases of CR and PR.
Fig. 3 shows variations in interferon γ (IFN γ) for the cases of PD.
Fig. 4 shows the relation between sugar chain structure and immune activity.
Fig. 5 shows the measured results of immune function of a steroid-administered case (Clinical Example 7) and various tumor markers.
Fig. 6 shows the measured results of immune function of a steroid-administered case (Clinical Example 8) and various tumor markers.
Fig. 7 shows the measured results of immune function of a steroid-administered case (Clinical Example 9) and various tumor markers.

### Best Mode for Carrying Out the Invention

The present invention will be illustrated in detail as follows and the technical and scientific terms used in the present specification have the meanings which are normally understood by persons having common knowledge in the technical field to which the present invention belongs unless otherwise defined.

The present invention has been done by investigating the correlation between clinical effect and cytokine. Here, the present inventor used a substance derived from mushroom mycelia and neovascularization inhibiting substance (cartilage of shark) jointly to patients suffering from progressive terminal cancer as a new immunotherapy (NITC) and measured various cytokines such as IL-12, IFN γ and IL-10. As a result, a strong positive correlation was found to be available in ratios of Th1/Th2 ratio to IL-12, Th1/Th2 ratio to IFN γ, IFN γ to IL-12 and IL-12 to CD 3 × CD 161 (NKR-P1) positive cells (CD 3+ CD 161+) and in a ratio of IFN γ to CD 3 × CD 161 (NKR-P1) positive cells and a strong reversed correlation was found to be available in a ratio of IL-12 to Vα24Vβ11 positive cells (Vα4+Vβ11+). Further, it was proved that NKT cells where Vα24Vβ11.T cell antigen receptor was stimulated showed a strong reversed correlation to the production amount of IL-12 and also showed a weak reversed correlation to IFN-γ production amount and to Th1/Th2 ratio and it was established that stimulation to Vα24Vβ11 acted as a suppression to the immune function. It was presumed that stimulation to Vα24Vβ11 may induce a large production of interleukin 4 (IL-4) and act on cellular immune suppression.

It was on the other hand proved that, when NK cell antigen receptor NKR-P1 of NKT cells was stimulated, the NKT cells showed a positive correlation to IL-12 and IFN-γ and showed a weak positive correlation to Th1/Th2 as well and it was established that stimulation to NKR-P1 acted as activation of immune function.

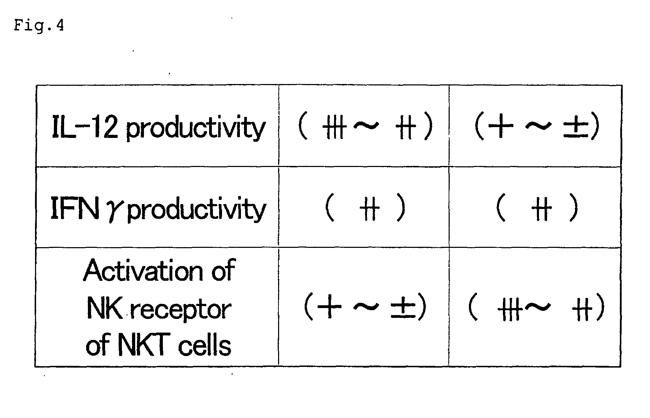

However, the substance derived from mycelia used in the above investigations contained a saccharide comprising a β1→3/1→6 steric structure and its action for activation of NKT cells was not always sufficient. The present inventor screened various candidate compounds and, as a result, a saccharide having an α1→3 steric structure very selectively and strongly acted on NKR-P1 (Fig. 4) whereupon the present invention has been achieved.

Thus, in screening a substance having an activating ability to NKT cells, it is necessary that at least the action to NKR-P1 is used an index to select a compound having an α1→3 steric structure and, further, it is preferred to conduct the selection using the fact that the said action is selective to NKR-P1 which is an NK cell antigen receptor in activation of NKT cells as an index. In addition, it is important that the said action does not affect Vα24Vβ11. As a result of the selective action of a substance selected as such, production of IFN γ in a large amount is induced and it is also possible that, in immune response, the immune system is induced in the direction where Th1 acts whereupon it is now possible to provide a very useful therapeutic agent for a cancer immunotherapy by the use of the said selected substance. In addition, the utility of this useful substance can be tested by checking whether it stimulates the NKR-P1-retaining cells, in the other word the cell having CD 3 × CD 161 which is a cell surface marker when the said substance is administered to organism.

The present invention provides a composition which contains an effective amount of a saccharide substance of an α1→3 steric structure having an ability of activating the NKT cells by a selective action to NKR-P1 of the above NKT cells.

With regard to a saccharide substance of an α1→3 steric structure having an ability of activating the NKT cells by a selective action to NKR-P1 of the NKT cells, there may be exemplified nigero-oligosaccharide (TSO), fucoidan and sulfated oligosaccharide etc.

Nigero-oligosaccharide is a saccharide containing 3-O-α-D-glucopyranosyl-D-glucose as a constituting unit. Representative examples thereof are nigerose (chemical formula 1), nigerosylglucose (chemical formula 2) and nigerosylmaltose (chemical formula 3) as shown below.

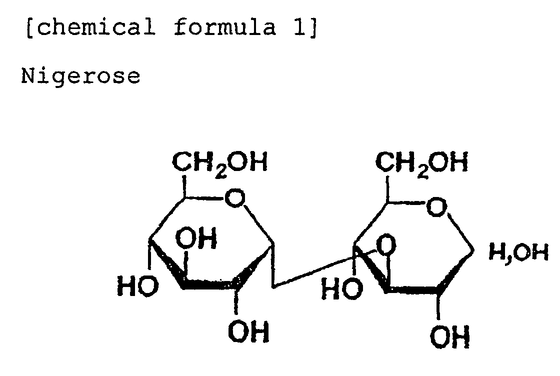

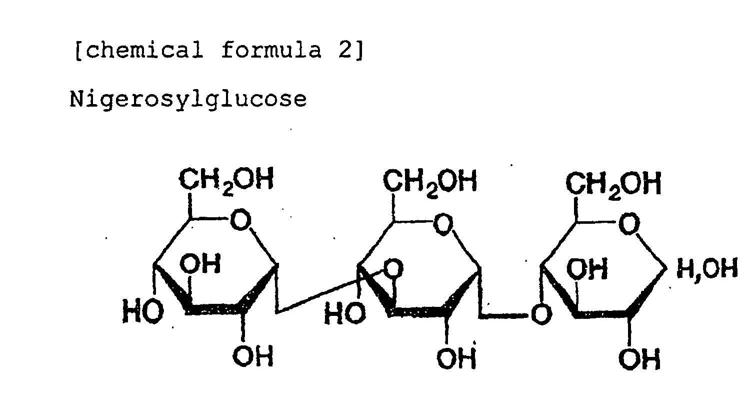

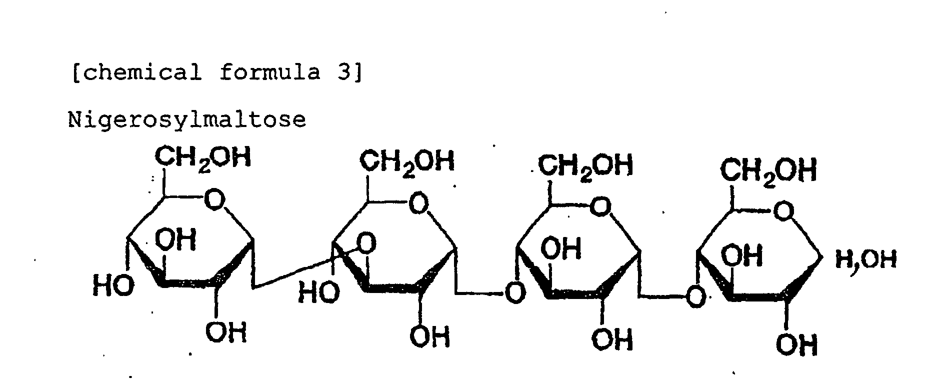

With regard to a commercially available nigero-oligosaccharide, there may be exemplified nigero-oligosaccharide liquid sugar (sold by Takeda Food Industry Co., Ltd.) and main nigero-oligosaccharides contained therein are ① nigerose α-D-Glc *p*-(1→3)-D-Glc, ② nigerosylglucose α-D-Glc *p*-(1→3)- α-Glc *p*-(1→4)-α-D-Glc and ③ nigerosylmaltose α-D-Glc *p*-(1→3)- α-Glc *p*-(1→4)-α-D-Glc *p*-(1→4)-D-Glc (in which Glc and *p* are abbreviations for glucose and pyranose, respectively).

In a narrow sense, fucoidan is a sulfate fucose-containing polysaccharide where one molecule of sulfuric acid is bonded to 2 to 6 molecules of fucose and a fucoidan-like polysaccharide where fucoidan further contains xylose or uronic acid is called "fucoidan" in a level of foods. Fucoidan is made into a preparation, for example, in such a manner that sea tangle is ground and made into chips, water-soluble components are extracted therefrom, the residue after extraction is removed by centrifugal separation and lower-molecular substances such as iodine and sodium chloride are removed by ultrafiltration followed by freeze-drying.

Examples of fucoidan are fucoidan derived from brown algae such as fucoidan derived from *Kjellmaniae crassifolia* and fucoidan derived from *Okinawa mozuku* (a kind of seaweed of the family *Spermatochnaceae*). In fucoidan derived from brown algae *Laminariaceae* such as *Kjellmaniae crassifolia*, there are contained at least three kinds of fucoidans, i.e. F-fucoidan (polymer of α-L-fucose), U-fucoidan (in which β-D-glucuronic acid and α-D-mannose are main chains and there is α-L-fucose in side chain) and G-fucoidan (in which β-D-galactose is a main chain and there is α-L-fucose in side chain) and, in any of the fucoidans, fucose is sulfated. As hereunder, structures of F-fucoidan (chemical formula 4), U-fucoidan (chemical formula 5) and G-fucoidan (chemical formula 6) of fucoidans derived from *Kjellmaniae crassifolia* (Takara Shuzo) will be shown. With regard to the structure of fucoidan derived from *Okinawa mozuku* belonging to family of *nagamatsumo*, brown algae, that manufactured by Takara Shuzo (chemical formula 7) and that manufactured by Morishita Jintan (chemical formula 8) will be shown below.

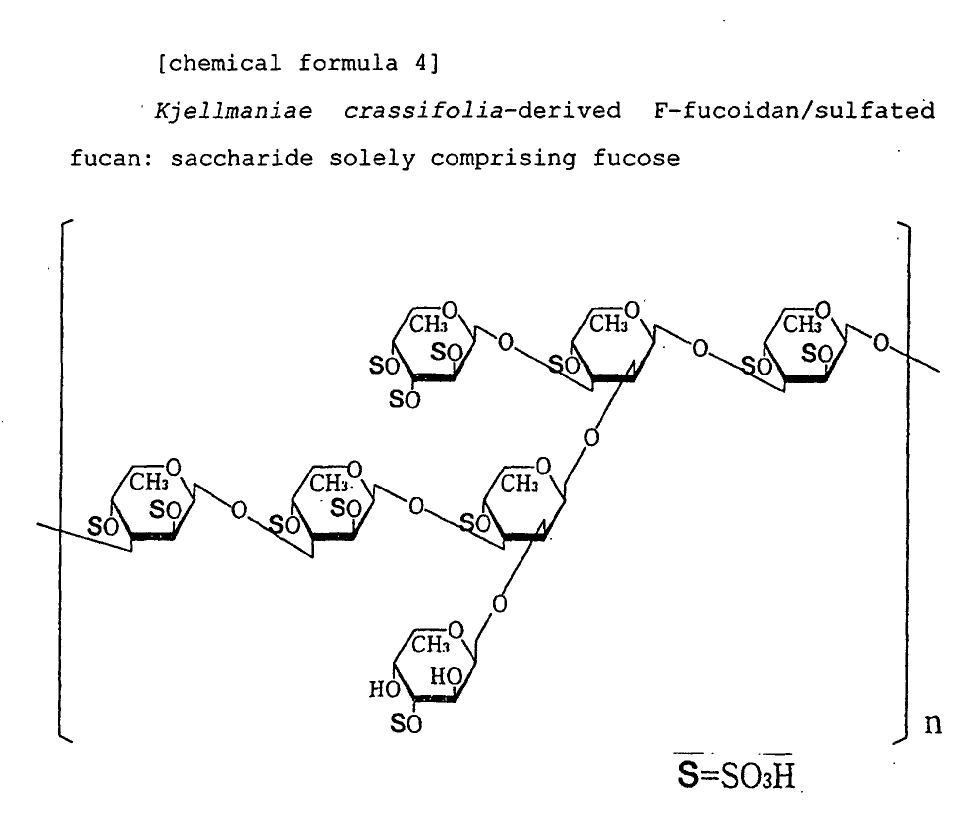

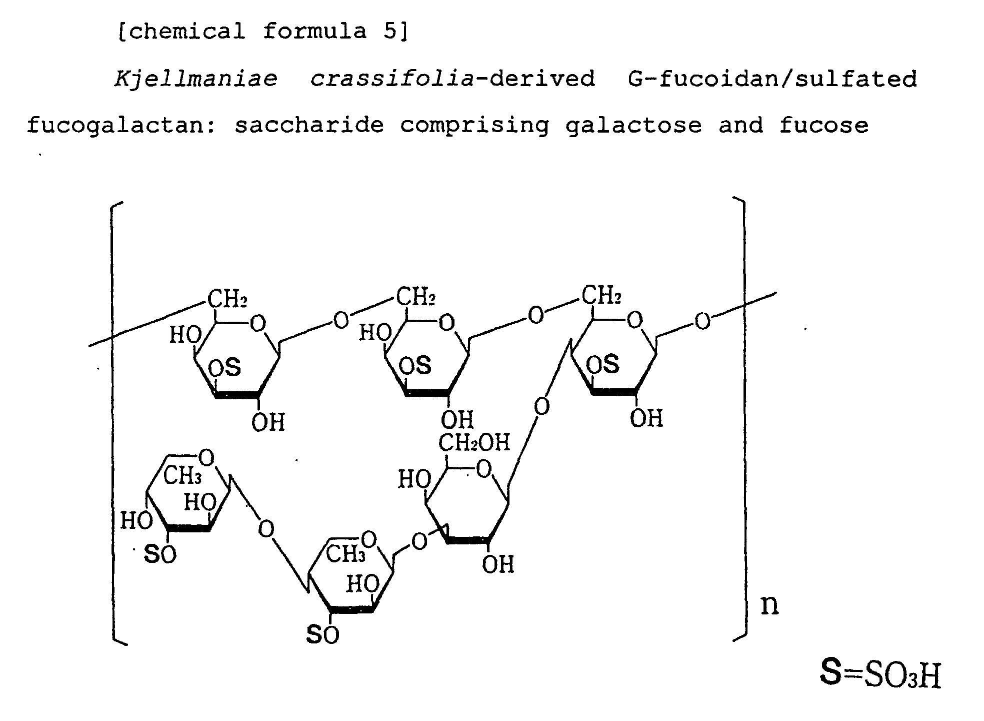

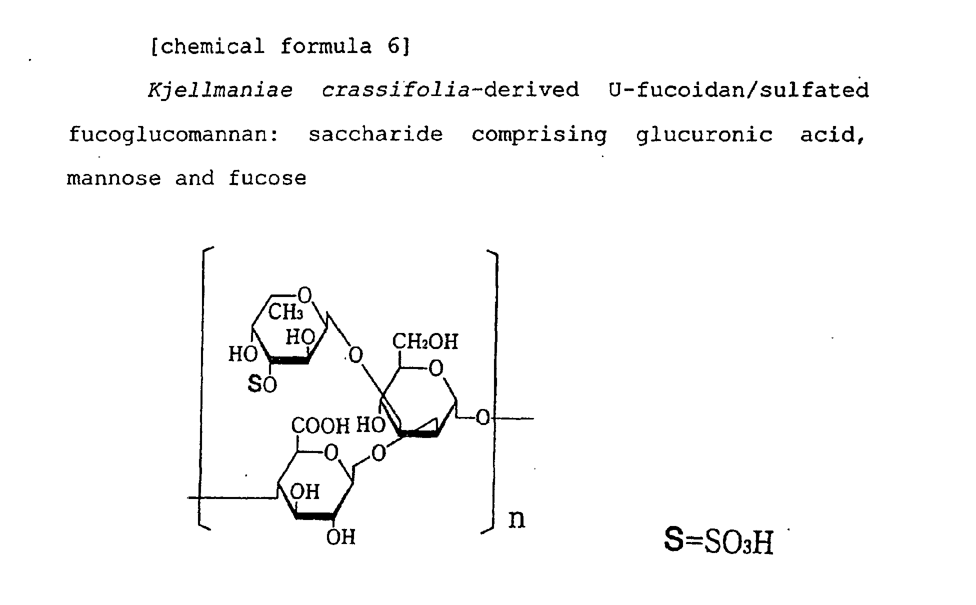

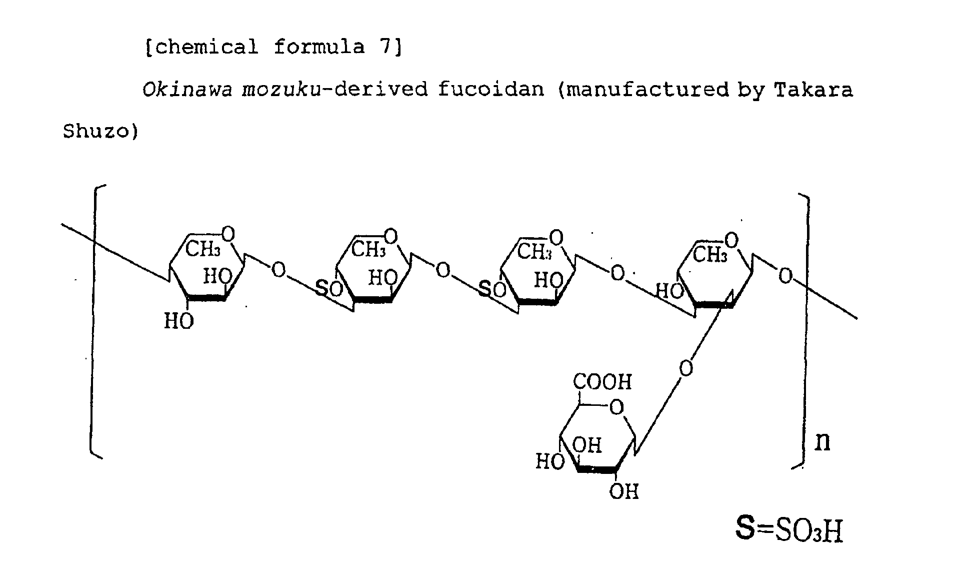

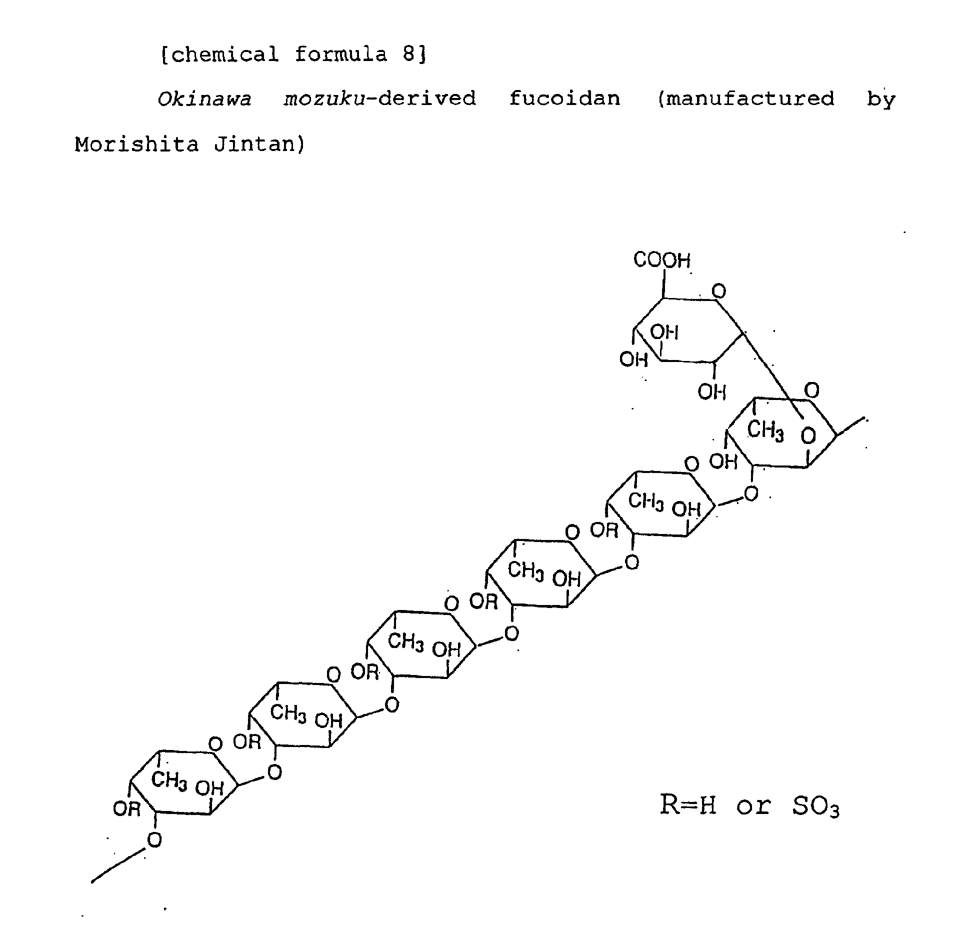

With regard to a sulfated oligosaccharide, there may be exemplified an extract derived from *Poryphyra Yezaensis* manufactured by K. K. Shirako. Main components for the said extract are an oligosaccharide of galactan sulfate of an α1→3 bond (chemical formula 9) and an oligosaccharide of galactan sulfate comprising an α1→3 bond and a β1→4 bond (chemical formula 10).

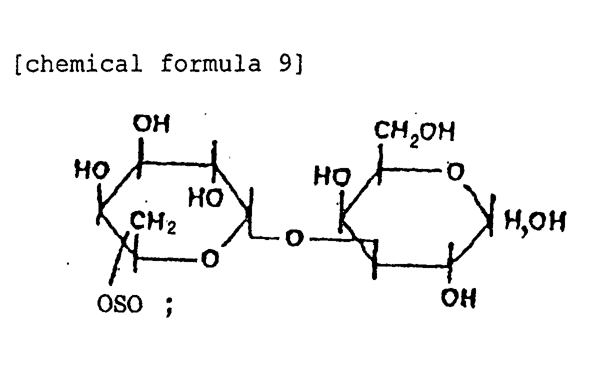

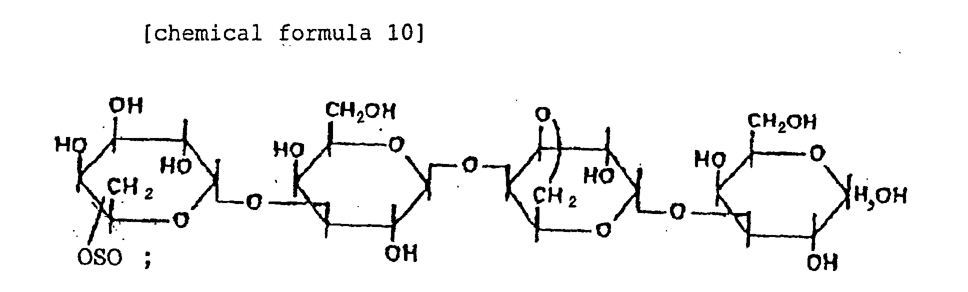

In the composition according to the present invention, at least one substance selected from the above-mentioned saccharide substances having an α1→3 steric structure is an active component. The said active component is not limited to those exemplified substances but may cover broad areas of substances which are saccharide substances having an α1→3 steric structure (saccharide component having an α1→3 glucoside bond structure) and having an ability of activating the NKT cells selectively acting on NKR-P1 of NKT cells.

The substance which activates the NKT cells by a selective action on NKR-P1 may be a composition containing a polysaccharide having an α1→3 steric structure and/or having 2∼10 oligosaccharides.

The composition where the above-mentioned saccharide substance having an α1→3 steric structure according to the present invention may be used as a therapeutic agent for cancer.

The said therapeutic agent for cancer is effective for the therapy of lung cancer (lung squamous carcinoma, lung adenocarcinoma and small-cell lung cancer), thymoma, thyroid cancer, prostatic cancer, renal cancer, bladder cancer, colon cancer, rectum cancer, esophageal cancer, cecum cancer, ureteral cancer, breast cancer, uterine neck cancer, brain tumor, cancer of the tongue, pharyngeal cancer, nasal cavity cancer, laryngeal cancer, cancer of stomach, hepatic cancer, cholangioma, testicular cancer, ovarian cancer, cancer of uterine body, metastatic bone cancer, malignant melanoma, osteosarcoma, malignant lymphoma, plasmacytoma, liposarcoma and the like although the present invention is not limited those cancers.

The composition or the therapeutic agent for cancer in accordance with the present invention is used in a formulation where the action on NKR-P1 of NKT cells in the activating ability of NKT cells is used as an index whereby the activation is induced or enhanced and the activation is further sustained. Thus, the above-mentioned composition and therapeutic agent for cancer is used by selecting the dose and the administering period where the action on NKR-P1 of NKT cells in the'activating ability of NKT cells is used as an index whereby the activation is induced or enhanced and the activation is further sustained. To be more specific, the dose is about 10 g to 40 g per day or, preferably, about 10 g to 20 g per day. The administering period is usually from 10 days to 24 months while the administering frequency is once to three times a day and, preferably, administration is done every day. The said composition or therapeutic agent for cancer is preferably given *per os*. It is of course also possible that the dose is reduced and they are prepared in a quality of being durable for parenteral administration whereby they are administered parenterally (including intravenous and intramuscular administrations).

In addition to the composition containing an effective amount of a saccharide substance of an α1→3 steric structure which is able to activate the NKT cells by a selective action on NKR-P1 of NKT cells, the above-mentioned therapeutic agent for cancer may further contain an effective amount of a composition which is able to induce the production of IL-12.

The composition of an effective amount of the above saccharide substance having an α1→3 steric structure according to the present invention may also be provided as a supplementary food preparation for health by oral administration which can be expected to have an anticancer effect as a result of administration. The said supplementary food preparation for health by oral administration may further contain an effective amount of a composition which is able to induce the production of IL-12.

The preparation for oral administration is prepared in tablets, diluted powder, capsules, syrups, etc. The preparation may be of course prepared by compounding with known and necessary additives such as filler, disintegrating agent, binder or lubricant and subjecting to a common means. If necessary, it is also possible to further add corrigent, colorant, flavor, stabilizer, bactericide, antiseptic agent, etc. thereto.

Another embodiment of the present invention relates to a new use of a composition of the saccharide substance having an α1→3 steric structure according to the present invention concerning application to each diseases. Two immune systems are participated in anti-tumor immunological competence and one of them is ① a system of TNF α (tumor necrosis factor α) → IFN γ → IL-12 → killer T cells while another is ② a system of NKT cell activation → perforin (cancer cell perforating factor). In the new immunotherapy (NITC) up to now, therapeutic results with nearly the same degree have been noted for those two systems. Thus, examples where the therapeutic effect was achieved as a result of activation of a system of ① IL-12 → killer T cell activation → apoptosis was activated and other examples where the therapeutic effect was achieved as a result of activation of a system of ② NKT cell activation → perforin → apoptosis was activated were noted about one half for each. However, when anticancer agent, radioactivity or joint steroid therapy was carried out, it has been firstly found that, one of the above-mentioned two immune systems, the system of TNF α → IFN γ → IL-12 → killer T cells is significantly inhibited. On the other hand, it has been newly found that the system of NKT cell activation → perforin is not inhibited at all.

Another embodiment of the present invention has been achieved by newly recomposing a cancer therapy method on the basis of the above phenomena.

Thus, when anticancer agent, radioactivity or joint steroid therapy is integrated into the therapy of cancer, the joint therapy is possible and the therapeutic effect becomes good when the immune system of ② is strong. However, when the immune system of ② is weak while only the immune system of ① is strong, then the joint therapy is presumed to result in failure. In that case, it is necessary to administer the α1-3 saccharide (a saccharide substance having an α1→3 steric structure) which activates NKT cells according to the present invention or, in other words, to jointly use the α1→3 saccharide which activated the NK receptor of NKT cells potentiating the immune system of ②. Alternatively, it is inevitable to adopt a low-concentration chemotherapy which is an administration method which does not inhibit the immune system of ① or, in other words, to adopt an administration method using low concentration of 5FU, UFT, Mifurol, Furtulon or CDDP (5 µg ∼ 10 µg) or low-concentration anticancer agent such as Taxotere™ or Taxol, adriamycin, mitomycin, CPT-11, etc. Similarly, it is necessary to adopt a low-dose irradiation in the radiotherapy and also to select a low-concentration administration method in the steroid therapy.

Therefore, when anticancer agent, radioactivity or steroid therapy is carried out, it is inevitable to measure various immunological competences for the object to be subjected to such a therapy. On the basis of the result of the measurement, it is necessary to administer a substance having an activating ability for NKT cells, in other word, a saccharide substance having an α1→3 steric structure when the immune system of ② is strong while for sustaining the immunological competences. When the immunity of the system of ② is weak, it is necessary to administer a saccharide substance having an α1→3 steric structure either in a large amount or directly into body such as administration by means of injection. When only the immunity of the system of ① works, one should adopt a method of administering an anticancer agent in such an amount that the immune system of ① is not inhibited, that is a low-concentration administration or adopt a method of quickly arousing the immune system of ① by administration of an anticancer agent whereby it is necessary to enhance the immune system of ①, that is to administer an IL-12-inducing substance in a large dose.

Further, when a composition where the above-mentioned saccharide substance having an α1→3 steric structure is a main component is used for the therapy of cancer, it is possible to judge the effectiveness of the said composition by means of a test where the action on NKR-P1 which is a natural killer (NK) cell antigen receptor of NKT cells in the activating ability of natural killer T (NKT) cells is used as an index. At that time, activation of NKT cells by a selective action of NKT cells to NKR-P1 can be tested by measuring the NKR-P1 by the measurement of CD 3 and CD 161 which are cell surface markers.

A commercial method in which the above-mentioned test means is used as a supplementary means for the therapy of cancer in liaison with medical organizations is also covered within a scope of the present invention.

As fully mentioned hereinabove, the present invention provides a composition containing a saccharide substance having an α1→3 steric structure which selectively acts on NKR-P1 of NKT cells to activate the NKT cells and also clarifies the relation between the ability of inducing the IL-12 production and the ability of activating the NKT cells by a selective action to NKR-P1 of NKT cells and, therefore, when those are carried on a commercial medium, that is a discriminating means for the value of the said product. Accordingly, a commercial medium carrying such information has a very high utility. The above-mentioned commercial medium means printed matters such as pamphlet, booklet or publication, magnetic recording medium such as floppy disk (FD), MO or CD-ROM, information transmitting medium to broad areas such as internet, and the like. Moreover, when such information is commercially utilized, that becomes a discriminating means for the value of the said product and, therefore, the commercial method utilizing the information is of very high utility.

Methods for the measurement of cells and each cytokine will be exemplified as hereunder.

### (Measurement of NKT cells)

Measurement of NKT cells having NKR-P1 can be carried out by measuring the cell surface antigen (CD 3 and CD 161) which are specifically present on the cell surface of NKT cells. To be specific, with regard to lymphocytes in peripheral blood, the cell where CD 3 is positive and CD 161 is also positive (CD 3+ CD 161+) is tested. Thus, CD 3 and CD 161 which are cell surface antigens of NKT cells are measured by a two-color test using a flow cytometry by the use of monoclonal antibody. Here, the thing that the NKT cells are activated means that, in the lymphocytes, the ratio of CD 3+ CD 161+ cells is not less than 10% or, more preferably, not less than 16%. The ability of activating the NKT cells means a function where the rate of NKT cells is increased to an extent of not less than 10% or, more preferably, not less than 16% or a function where the rate of NKT cells before administration of a certain substance is further increased.

In the Examples, blood of patients suffering from caner was used and the rate of the cells where CD 3+ and CD 161+ which are cell surface antigens in the cells in blood was measured by way of a common method by a two-color test using a flow cytometry. As to the monoclonal antibodies to CD 3 and to CD 161 at that time, there were used CD3-PC5 manufactured by Coulter and CD 161 manufactured by Becton Dickinson, respectively.

Measurement of Vα24Vβ11·NKT cells can be carried out by measuring the cell surface antigen (Vα24 and Vβ11) which are specifically present on the cell surface of NKT cells. To be specific, with regard to lymphocytes in peripheral blood, the cell where Vα24 is positive and Vβ11 is also positive is tested. Thus, Vα24 and Vβ11 which are cell surface antigens of NKT cells are measured by a two-color test using a flow cytometry by the use of monoclonal antibody (TCR-Vα24PE, TCR-Vβ11FITC) (manufactured by Beckman Coulter).

### (Measurement of Perforin-Producing Cells)

With regard to lymphocytes in peripheral blood, the rate of cells where CD 3 and CD 161 are positive and perforin is also positive is measured according to a common method by a three-color test using a flow cytometry. To be specific, a fixing solution is added to the collected blood to fix the cells, a liquid permeating through a membrane is added, then reaction is carried out by adding an anti-perforin antibody (manufactured by Pharmingen), a PRE-Cy5 labeled secondary antigen (manufactured by DAKO) is further added to react, then reaction is carried out by adding an anti-CD3-PE (Coulter 6604627) and an anti-CD161-FITC (B-D) thereto and, after that, measurement is carried out by a flow cytometry.

### (Preparation of Sample for Measurement of Cytokine)

Firstly, a mono nuclear cell fraction is separated and prepared from blood. Heparin-added peripheral blood is diluted to an extent of 2-fold by a phosphate-buffered saline (PBS) followed by mixing, layered on a Ficoll-Conray liquid (specific gravity: 1.077), a centrifugal precipitation is carried out at 400 G for 20 minutes and a mono nuclear cell fraction is collected. After washing, an RPMI-1630 medium to which 10% fetal bovine serum (FBS) are added is added thereto to prepare so as to make the cell numbers 1 × 10⁶. To 200 µl of the resulting cell floating liquid is added phytohemagglutinin (manufactured by Difco) to make its concentration 20 µg/ml and the mixture is incubated at 37°C for 24 hours on a 96-well microplate in the presence of 5% of CO₂ to prepare a sample for measurement of cytokine in the said incubated cell solution.

### (Measurement of IL-12)

In the measurement of the amount of IL-12, although clinical and biochemical tests which have been known *per se* may be utilized, there may be used measuring kits by an enzyme immunoassay (ELISA) which are available from R&D Systems and MBL. Here, a measuring kit of R&D Systems was used. Thus, 50 µl of the Assay Diluent RD1F and 200 µl of standard liquid or the sample prepared in Example 1 were placed in each well of a 96-well microplate and made to react for 2 hours by allowing to stand at room temperature. After that, 200 µl of anti-IL-12 antibody labeled with horse radish peroxidase (HRP) were placed to each well followed by being allowed to stand for 2 hours at room temperature. The reaction solution in each well was removed and washed for three times, each 200 µl of a colorizing substrate solution were added, the mixture was allowed to stand for 20 minutes at room temperature and each 50 µl of a solution for stopping the enzymatic reaction were added thereto. Absorbance of each well at 450 nm was measured by an Emax (manufactured by Wako Pure Chemicals) using that at 550 nm as a control. Amount of IL-12 is expressed in terms of pg/ml. Here, the ability of inducing the production of IL-12 means a function where the amount of IL-12 produced by the stimulation of peripheral mono nuclear cell fraction is enhanced to an extent of not less than 7.8 pg/ml or a function where the amount of production of IL-12 is enhanced than that before administration of a certain substance.

### (Measurement of IFN γ)

Measurement of IFN γ was conducted by an enzyme immunoassay (EIA) using an IFN γ EASIA Kit of BioSource Europe S. Thus, 50 µl of a standard solution or the above-prepared sample which was diluted to an extent of 2-fold were placed in each well of a 96-well microplate, each 50 µl of HRP-labeled anti-IFN-γ antibody were placed and the reaction was carried out with shaking for 2 hours at room temperature. The reaction solution in each well was removed, washed for three times, each 200 µl of a coloring substrate solution were added, the reaction was carried out with shaking for 15 minutes at room temperature and each 50 µl of a stop solution for enzymatic reaction were placed. Absorbances of each well at 450 nm and 490 nm was measured by an Emax (manufactured by Wako Pure Chemical) using that at 630 nm as a control. The amount of IFN γ was expressed in terms of IU/ml.

### (Measurement of IL-10)

Measurement of IL-10 was conducted by a solid-phase enzyme immunoassay (ELISA) using an IL-10 EASIA Kit of BioSource Europe S. The method was carried out according to the method for the measurement of IFN γ except the use of anti-IL-10 antibody. Amount of IL-10 was expressed in terms of pg/ml.

### (Measurement of Cellular Ratio of Th1/Th2)

The cellular ratio of Th1/Th2 was tested by a conventional method by means of a helper T (Th) cellular system three-color analytical test by a flow cytometry. Th1/Th2 means a ratio of cells which produce IFN γ (Th1) and cells which produce IL-4 (Th2) in helper T cells having a cell surface antigen CD 4.

Firstly, blood was treated with phorbol 12-myristate 13-acetate and ionomycin at 37°C for 4 hours whereby the cells in blood were stimulated to produce cytokine. Then breferdin A was added to stop the production reaction, CD 4-PC 5 (manufactured by Beckman Coulter) which was an anti-CD 4 antibody was used to stain CD 4 which was a cell surface marker, the cells were fixed and a hemolytic treatment was carried out using an FACS Lysing Solution (manufactured by Nippon Becton Dickinson). After that, a cell membrane permeation treatment was carried out using an FACS Permeabilizing Solution (manufactured by Nippon Becton Dickinson), then cytokine in the cells was stained with an anti-IFN γ antibody/anti-IL-4 antibody (FASTIMMUNE IFN γ FITC/IL-4 PE) (manufactured by Nippon Becton Dickinson) and measurement and analysis were carried out using a flow cytometer (FACS Calibur) (manufactured by Becton Dickinson).

### EXAMPLES

Clinical examples will be shown as examples of the present invention for further specific illustration although the present invention is not limited thereto but various applications are possible within such a range that they are not out of the technical idea of the present invention.

Each of various tumor markers measured in each clinical example was measured by a respective known mean. Figure given in the lower line for each test item in the table show the normal value for each item. Effectiveness of the therapy used is expressed in the table as completely cured (CR), partially cured (PC), no change (no progress of cancer) (NC) or invalid or progressive disease (PD) in accordance with the standard for judgment of the efficacy of anticancer agent under GCP of the Japan Ministry of Health and Welfare.

### (Clinical Example 1)

Nigero-oligosaccharide (TOG) was administered 12.0 g per day and an effective case of anticancer effect was achieved.

The patient was a female of 67 years age suffering from sigmoid cancer. At the first medical examination, although IL-12 was produced, the rate of NKT cells was 11.8% and productivity of perforin (PERF) of NKT cells was not more than 4.3% whereby the NKT cells were not activated. Although the mushroom mycelium component was continuously administered for 4 month thereafter, activation of the NKT cells was not achieved. However, since IL-12 was still produced, tumor markers of STN and ICTP did not rise whereby the case was judged to be NC.

After that, a daily dose of 12.0 g of TOG having an α1-3 glucan structure was administered by dividing into three times a day. Administration of TOG was carried out every day. As a result, after about 3 months, the rate of NKT cells significantly increased to 24.4% and productivity of perforin by the NKT cells also significantly increased to 6.5% whereupon activation of the NKT cells was noted. After 1 month more, STN and ICTP decreased to the normal values of 45 U/ml and 4.6 ng/ml, respectively and lymph node in abdominal cavity disappeared as well.

It is therefore likely that, as a result of administration of 12.0 g per day of TOG, levels of NKT cell numbers and perforin producing ability rose and tumor markers lowered whereupon the metastatic lymph node disappeared.

### (Clinical Example 2)

Sulfated oligosaccharide (manufactured by K. K. Shirako) was administered at the dose of 20.0 g per day and an effective case of anticancer effect was achieved.

A male of 55 years age suffering from multiple bone metastases to rib, thoracic vertebra, lumbar vertebra, etc. caused by unknown primary cancer was administered with mushroom mycelium component, shark cartilage and a daily dose of 20.0 g of sulfated oligosaccharide containing a large amount of α1-3 glucan (manufactured by K. K. Shirako) by dividing into three times a day. Administration of the sulfated oligosaccharide was conducted every day. Although IL-12 was rarely produced, values of various tumor markers were significantly improved. After 4 months, the rate of the NKT cells rose to 17.9% and their perforin productivity was activated to an extent of 4.7% whereupon the bone metastases and pain caused by them were significantly improved as well. During that period, IL-12 was rarely produced.

It is likely that improvements in bone metastases and pain caused by them in this case was due to increases in the NKT cell numbers and their perforin productivity by administration of 20 g per day of sulfate oligosaccharide (manufactured by K. K. Shirako).

### (Clinical Example 3)

Nigero-oligosaccharide (TOG) was administered at the dose of 12.0 g per day and an effective case of anticancer effect was achieved.

In a male of 73 years age suffering from lung adenocarcinoma, a new immunotherapy (NITC) (formulation of mushroom mycelium component and shark cartilage) was started as from the first medical examination. IL-12 was produced and, among the tumor markers, although NCC-ST-439 and SLX-1 lowered, CEA and Ca15-3 did not lower. Before administration of TOG at 8 months thereafter, a part of tumor markers lowered and, therefore, the case was judged to be PR. After that, a daily dose of 12.0 g of TOG was administered by dividing into three times a day. TOG was administered every day. After 2 months, the rate of NKT cells rose to 27.4% and perforin productivity of the NKT cells was also activated to 13.3%. As a result thereof, CEA and NCC-ST-439 were normalized, Ca15-3 and SLX-1 which were other tumor markers were significantly reduced to 82 U/ml and 59 U/ml, respectively as well and, in addition, improvement of the symptom was noted.

In this case, it is also likely that effectiveness of the cancer therapy was enhanced as a result of immunological enhancement by IL-12 production and immunological enhancement by an increase in NKT cell numbers and activation of function thereof.

### (Clinical Example 4)

Sulfated oligosaccharide (manufactured by K. K. Shirako) was administered at the dose of 20.0 g per day and an effective case of anticancer effect was achieved.

An NITC therapy was started to a male of 68 years age suffering from rectal cancer and hepatic metastasis. Until 5 months thereafter, among the tumor markers, no change was noted for Ca19-9 and ICTP while tendency of an increase in CEA was noted. From the fifth month, an every-day administration of 20.0 g per day of sulfated oligosaccharide (manufactured by K. K. Shirako) was started. In the second month thereafter, rate of the NKT cells rose to 22.2% and perforin productivity of the NKT cells was also activated to 7.8%. After that, the NKT cell activity continued similarly. During that period, all of the tumor markers were normalized resulting in CR.

In this case, although the IL-12 was produced even in the initial stage, the clinical improvement was NC. However, after the administration of sulfated oligosaccharide (manufactured by K. K. Shirako) at the dose of 20 g per day, there were achieved an increase in the NKT cell numbers and activation of the function thereof whereby the result was CR. Such a fact'was likely to be due to activation of the NKT cells by administration of the sulfated oligosaccharide (manufactured by K. K. Shirako) at the dose of 20 g per day.

### (Clinical Example 5)

U-Fucoidan (trade name; an oligosaccharide derived from sea tangle) was administered at the dose of 15.0 g per day whereupon an effective case of anticancer effect was achieved.

An NITC therapy was started to a female of 58 years age suffering from breast cancer, lung metastasis, lymph node metastasis and bone metastasis. Since neither NKT cell activation nor IL-12 production was achieved by that, administration of 15.0 g per day of U-fucoidan containing a large amount of α1-3 glucan was started after 4 months. Until that time, all of the tumor markers increased but, after 3 months from the start of the administration, rate of the NKT cells significantly rose to 21.1% and ability of NKT cells for the production of perforin was also activated to 6.6%. As a result, various tumor markers were reduced to an extent of about one half and pain and lack of appetite were improved as well resulting in PR.

Reduction in tumor markers and clinical improvement as such are likely due to an increase in the NKT cell numbers and activation of the function thereof by administration of 15.0 g per day of U-fucoidan.

### (Clinical Example 6)

Fucoidan derived from *Okinawa mozuku* was administered at the dose of 15.0 g per day and an effective case of anticancer effect was achieved.

An NITC therapy was started to a case of male of 69 years age suffering from ureteral cancer and prostatic cancer but, during two years and five months, the tumor markers slowly increased. Usual social life was however possible. As from two years and fifth month, administration of 15.0 g per day of fucoidan derived from *Okinawa mozuku* containing a large amount of α1-3 glucan was started. As a result, 3 months thereafter, an increase (17.5%) in the rate of NKT cells and an enhancement (5.7%) in ability of NKT cells for the production of perforin were achieved together with an enhancement (21.2 pg/ml) of IL-12 production. Various tumor markers were normalized, both ureteral cancer and bladder metastasis disappeared and prostatic cancer also disappeared whereby the judgment was CR.

Such a significant clinical improvement was achieved by administration of 15.0 g per day of fucoidan derived from *Okinawa mozuku.*

### (Test Example 1)

Variations in CD 3+ CD 161+ cells in the cases to which TOG was administered were investigated. Nigero-oligosaccharide (TOG) of 9.0 g to 18.0 g per day was additionally administered to 148 cancer-bearing cases to which an NITC was applied. Before the administration of TOG, the rate of NKT cells was 16.19 ± 7.25%. When the cases were checked as a whole, there was a tendency of an increase in the NKT cells until 1 to 6 month(s) after the administration but no significant difference was noted. However, in the effective cases resulting in CR and PR, the data before the administration were 16.95 ± 9.22% while, after 1 month from the administration, they significantly increased to 22.73 ± 11.08% (p < 0.05) and, even after that, a tendency for high data was noted. The NKT cells significantly increased (p < 0.01) even in the cases of PD. On the other hand, in the progressive cases of malignant tumor, although the NKT cell numbers tended to increase on the second month after the administration of TOG as compared with the data before the therapy, there was no tendency of a significant increase. It was also found that the cases where the ratio of the NKT cells was more than 16.0% were able to prolong their lives for a long period.

### (Test Example 2)

Variations in IL-12 in the cases to which TOG was administered were investigated. Nigero-oligosaccharide (TOG) of 9.0 g to 18.0 g per day was additionally administered to 148 cancer-bearing cases to which an NITC was applied. There was no big change in the additionally-administered cases as a whole. Although there was a tendency of an increase in the NK cell numbers until 1 to 6 month(s) from the administration, there was no significant difference. However, in the effective cases (45 cases) comprising CR and PR, production of IL-12 was significantly enhanced in the second month (p < 0.05). On the other hand, no enhancing effect for IL-12 was noted in the cases of PD (60 cases).

### (Test Example 3)

### Variations in IFN γ in the cases to which TOG was administered were investigated.

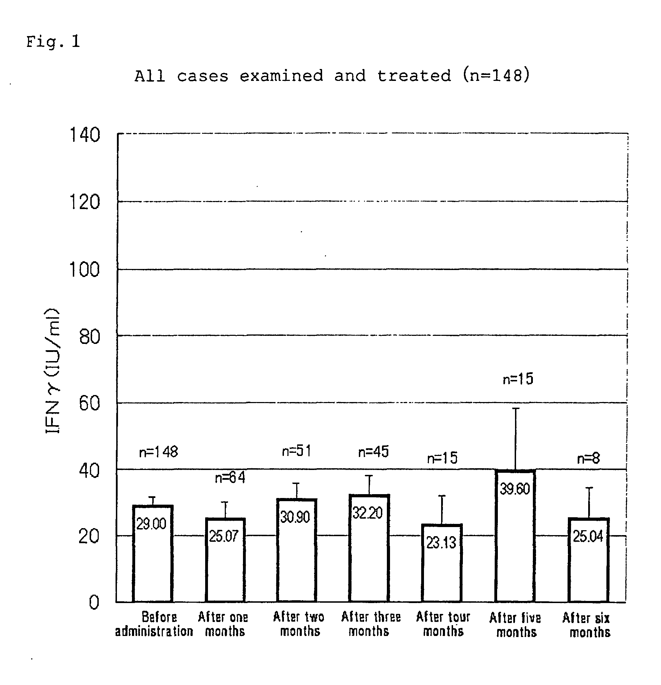

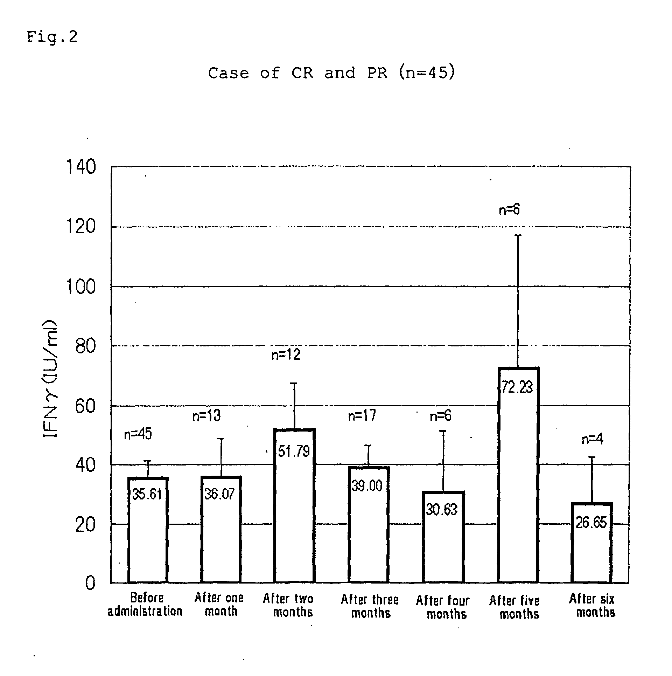

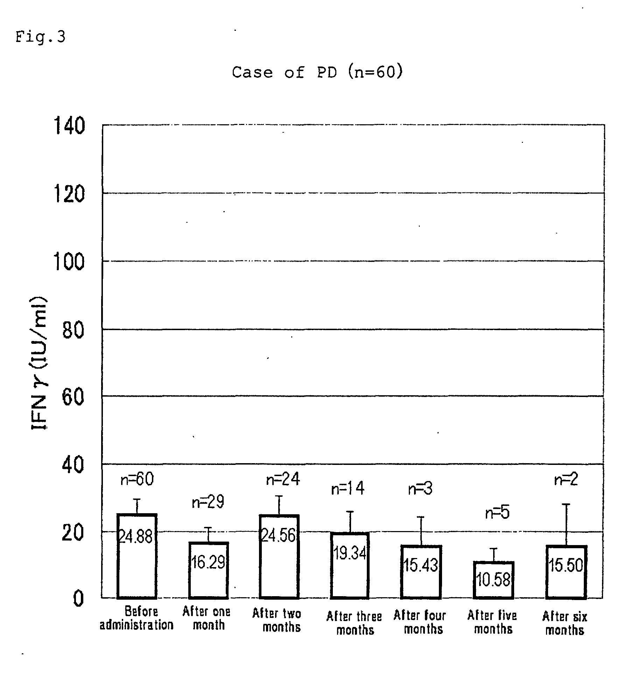

In 45 effective cases (CR and PR) among 148 cases to which TOG was administered, there were many examples where ability for the production of IFN γ was high while, in the cases of PD, there were many cases where it was low (refer to Figs. 1 to 3). Thus, in the effective cases by the administration of TOG, there were many cases where the high data of IFN γ was sustained. That fact suggests that TOG also has an action of sustaining the productivity of IFN γ.

### (Clinical Example 7)

### Clinical example where steroid (20 mg/day) was administered

In a case of terminal cancer of uterine cancer and breast cancer, platelets were significantly decreased to 21,000 mm³/ml resulting in a shock state and, therefore, 20 mg/day of prednisolone were administered. Further, in addition to an NITC (using a formulation of mushroom mycelium component and shark cartilage), the administration of fucoidan derived from *Okinawa mozuku* (15.0 g/day) was continued.

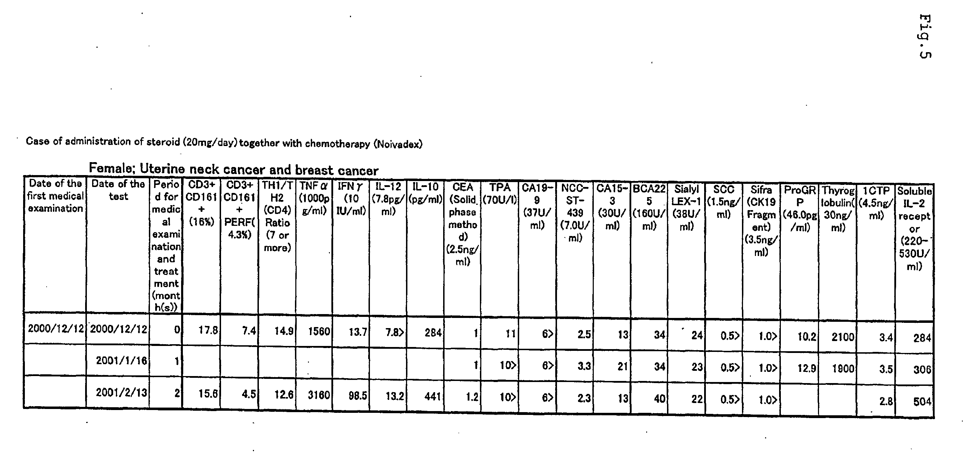

Rate of CD 3 + CD 161 + NKT cells increased to 17.8% at the first medical examination and its perforin producing ability (PERF) was activated to 7.4%. However, the IL-12 producing ability was lowered to 7.8 pg/ml or less. That is because the immune system ① (TNF α → IL-12 → killer T cell system) is suppressed by the steroid (Fig. 5).

### (Clinical Example 8)

### Clinical example where steroid (30 mg/day) was administered

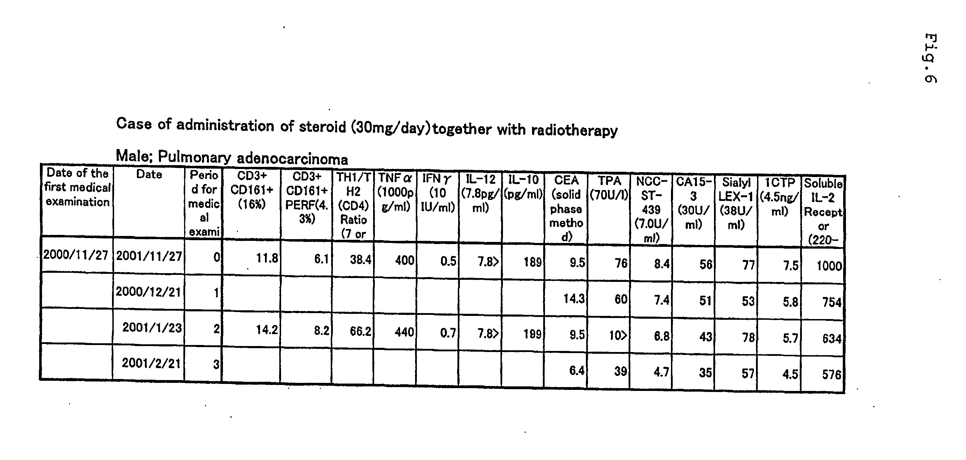

This is a case suffering from pulmonary adenocarcinoma and is receiving administration of 30 mg/day of steroid, radiotherapy, NITC therapy and administration of 15.0 g/day of U-fucoidan. Since the production of IL-12 is not noted in two measurements, it seems that the immune system ① (TNF α → IL-12 → killer T cell system) is suppressed. However, since both NKT cell numbers and perforin productivity thereof are noted, it was suggested that the immune system ② (NKT cell activation → perforin) to which NKT acts is sustained (Fig. 6).

### (Clinical Example 9)

### Case where steroid (20 mg/day) was administered

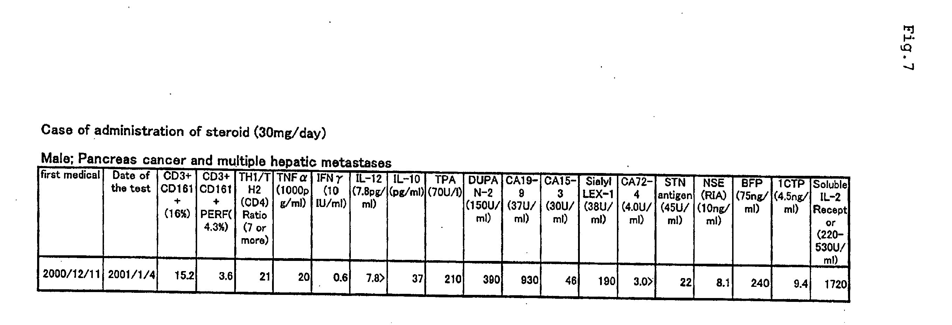

This is a case of terminal cancer suffering from pancreas cancer and multiple hepatic metastases and 20 mg/day of steroid, NITC therapy and 15.0 g/day of F-fucoidan derived from *Kjellmaniae crassifolia* were formulated. Productivity of IL-12 and TNF α participating in the immune system ① (TNF α → IL-12 → killer T cell system) significantly lowered. On the other hand, although the rate of NKT cells participating in the immune system ② (NKT cell activation → perforin) was in a somewhat lowering tendency(15.2%), it was still retained to some extent (Fig. 7).

### (Clinical Example 10)

### Case where 5FU and leucovorin are administered

This is a case suffering from colon adenocarcinoma and is receiving an NITC therapy and administration of 12.0 g/day of nigero-oligosaccharide (TOG). From 3.5 months after starting the NITC therapy and administration of TOG, there was also carried out the chemotherapy by 5FU and 600 mg/day of leucovorin. Although IL-12 productivity was 14.5 pg/ml before carrying out the chemotherapy, it became 7.8 pg/ml or less after the chemotherapy. However, the rate of CD 3+ CD 161+ NKT cell was sustained at 15.2% even during the chemotherapy. From the result, it is likely that the immune system ① (TNF α → IL-12 → killer T cell system) was suppressed by the chemotherapeutic agents. On the other hand, it was shown that the immune system ② (NKT cell activation → perforin) on which NKT acted was sustained.

### (Clinical Example 11)

### Case where UFT was administered

This is a case suffering from sigmoid colon cancer where an NITC therapy and administration of 15.0 g/day of U-fucoidan are applied from the fourth month after surgical excision of sigmoid colon. After one year and four months from the start of the NITC therapy and U-fucoidan administration, chemotherapy using UFT (suppositories; 400 mg/day) was carried out but it was ceased after 10 months. IL-12 productivity was sustained at 10 pg/ml or more with the highest data of 41.5 pg/ml before the chemotherapy, but it significantly lowered to 7.8 pg/ml or less during the course of chemotherapy. After ceasing from the chemotherapy however, the IL-12 productivity recovered. The rate of CD 3+ CD 161+ NKT cells was sustained at 10% or more during the chemotherapy and even after ceasing therefrom. From those results, it is likely that the immune system ① (TNF α → IL-12 → killer T cell system) was suppressed by the chemotherapeutic agent. On the other hand, it was shown that the immune system ② (NKT cell activation → perforin) on which NKT acted was sustained.

### (Clinical Example 12)

### Case where 5FU was administered

This is a case suffering from rectum cancer and receiving an NITC therapy and administration of 20.0 g/day of sulfate oligosaccharide (manufactured by K. K. Shirako). After one month from the start of the NITC therapy and the administration of sulfated oligosaccharide, chemotherapy using 500 mg/2 weeks of 5FU was also carried out. The IL-12 productivity was 13.1 pg/ml before conducting the chemotherapy but it lowered to 7.8 pg/ml or less after the chemotherapy was started. The rate of CD 3+ CD 161+ NKT cell was sustained at 13% to 15% even during the chemotherapy. From those results, it is likely that the immune system ① (TNF α → IL-12 → killer T cell system) was suppressed by the chemotherapeutic agent. On the other hand, it was shown that the immune system ② (NKT cell activation → perforin) on which NKT acted was sustained.

### (Clinical Example 13)

### Case where CDDP, 5FU and Endoxan were administered

This is a case suffering from breast cancer and receiving an NITC therapy and administration of 15.0 g/day of fucoidan derived from *Okinawa mozuku.* From the fifth month of the NITC therapy and the administration of fucoidan derived from *Okinawa mozuku*, chemotherapy by administration of CDDP, 5FU and Enxodan in four cycles was also carried out. Although the IL-10 productivity increased to 38.3 pg/ml before the chemotherapy was carried out, it significantly lowered to 7.8 pg/ml or less after the chemotherapy was started. The rate of CD 3+ CD 161+ NKT cell was sustained at around 19% even during the chemotherapy. From those results, it is likely that the immune system ① (TNF α → IL-12 → killer T cell system) was suppressed by the chemotherapeutic agent. On the other hand, it was shown that the immune system ② (NKT cell activation → perforin) on which NKT acted was sustained.

### (Clinical Example 14)

### Case where CDDP and Taxotere™ were administered

This is a case suffering from pulmonary squamous carcinoma and receiving an NITC therapy and administration of 15.0 g/day of U-fucoidan. After 1.5 months from the NITC therapy and the administration of U-fucoidan, chemotherapy by administration of CDDP and Taxotere™ in three cycles was also carried out. Although the IL-12 productivity increased to 229 pg/ml before the chemotherapy was carried out, it significantly lowered to 7.8 pg/ml or less after the chemotherapy was started. The rate of CD 3+ CD 161+ NKT cell was 8.1% before the chemotherapy but, after carrying out the chemotherapy, it became 9.1% and, although it was less than 10%, no decrease was noted but some increase was rather noted. From those results, it is likely that the immune system ① (TNF α → IL-12 → killer T cell system) was suppressed by the chemotherapeutic agent. On the other hand, it was shown that the immune system ② (NKT cell activation → perforin) on which NKT acted was sustained.

### (Clinical Example 15)

### Case of Radiotherapy

This is a case suffering from liver cancer and receiving an NITC therapy and administration of 12.0 g/day of nigero-oligosaccharide (TOG). From one month since the start of the NITC therapy and the TOG administration, metastasis to frontal brain was noted and, therefore, radiotherapy was started to the said metastatic site and continued for about three weeks. Although the IL-12 productivity was 60.3 pg/ml before the radiotherapy was carried out, it significantly lowered to 8.5 pg/ml after the radiotherapy was started. The rate of CD3+ CD161+ NKT cell was 11.1% before the radiotherapy but, after carrying out the radiotherapy, it increased to 13.7%. From those results, it is likely that the immune system ① (TNF α → IL-12 → killer T cell system) was suppressed by the radiotherapy. On the other hand, it was shown that the immune system ② (NKT cell activation → perforin) on which NKT acted was sustained.

### (Clinical Example 16)

### Case of Radiotherapy

This is a case suffering lung cancer and receiving an NITC therapy and administration of 15.0 g/day of U-fucoidan. Radiotherapy was started nearly at the same stage as the start of the NITC therapy and the U-fucoidan administration and, after about 2.5 months, the radiotherapy was ceased. Although the IL-12 productivity was 7.8 pg/ml or less during the radiotherapy, it significantly increased to 46.5 pg/ml after two months from ceasing from the radiotherapy. The rate of CD3 + CD161 + NKT cell was sustained at 10% or more both during the radiotherapy and after ceasing from it. From those results, it is likely that the immune system ① (TNF α → IL-12 → killer T cell system) was suppressed by the radiotherapy. On the other hand, it was shown that the immune system ② (NKT cell activation → perforin) on which NKT acted was sustained.

### (Clinical Example 17)

### Case of Radiotherapy

This is a case suffering from breast cancer and receiving an NITC therapy and administration of 12.0 g/day of nigero-oligosaccharide (TOG). From 5.5 months since the start of the NITC therapy and the TOG administration, metastases to bone and brain were noted and, therefore, radiotherapy was started to the said metastatic sites. Although the IL-12 productivity was 9.4 pg/ml before the radiotherapy was carried out, it lowered to 7.8 pg/ml after the radiotherapy was started. The rate of CD 3+ CD 161+ NKT cell was 17%∼19% even during the radiotherapy. From those results, it is likely that the immune system ① (TNF α → IL-12 → killer T cell system) was suppressed by the radiotherapy. On the other hand, it was shown that the immune system ② (NKT cell activation → perforin) on which NKT acted was sustained.

### (Clinical Example 18)

### Case of Radiotherapy

This is a case suffering cholangiocarcinoma and receiving an NITC therapy and administration of 20.0 g/day of sulfated oligosaccharide (manufactured by K. K. Shirako). Radiotherapy was started nearly at the same stage as the start of the NITC therapy and the sulfated oligosaccharide administration and, after about one month, the radiotherapy was ceased. Although the IL-12 productivity was 18.2 pg/ml before carrying out the radiotherapy, it lowered to 9.8 pg/ml during the radiotherapy but it increased to 32.9 pg/ml after four months from ceasing from the radiotherapy. The rate of CD 3+ CD 161+ NKT cell was 11%∼21% even during the radiotherapy. From those results, it is likely that the immune system ① (TNF α → IL-12 → killer T cell system) was suppressed by the radiotherapy. On the other hand, it was shown that the immune system ② (NKT cell activation → perforin) on which NKT acted was sustained.

### (Clinical Example 19)

### Case of Radiotherapy

This is a case suffering from pulmonary adenocarcinoma and receiving an NITC therapy and administration of 15.0 g/day of fucoidan derived from *Okinawa mozuku.* From the third month of the NITC therapy and the administration of fucoidan derived from *Okinawa mozuku*, metastases to brain and lumbar vertebra were noted and, therefore, radiotherapy was started to the said metastatic sites. Although the IL-12 productivity before carrying out the radiotherapy was 57.7 pg/ml or more, it lowered to 7.8 pg/ml after the radiotherapy was started. The rate of CD 3+ CD 161+ NKT cell before carrying out the radiotherapy was 17.2% and, during the radiotherapy, it was still 12.8%. From those results, it is likely that the immune system ① (TNF α → IL-12 → killer T cell system) was suppressed by the radiotherapy. On the other hand, it was shown that the immune system ② (NKT cell activation → perforin) on which NKT acted was sustained.

### (Clinical Example 20)

### Case of Chemotherapy, Radiotherapy and Steroid Administration

This is a case suffering from lung cancer and receiving an NITC therapy and administration of 12.0 g/day of nigero-oligosaccharide (TOG). Chemotherapy by administration of 69 mg of Taxotere™ and 49 mg of cisplatin was started nearly at the same stage as the NITC therapy and the TOG administration were started. After about 3 months, the chemotherapy was ceased but, since brain metastasis was noted at 2.5 months from the ceasing, radiotherapy using a gamma knife was carried out. After more one weak, radiotherapy was conducted again and administration of steroid was carried out for ten days. Although the IL-12 productivity was 7.8 pg/ml or less during the chemotherapy, the radiotherapy and the steroid administration, it increased to 65.5 pg/ml when those therapies were ceased. The rate of CD 3+ CD 161+ NKT cell was 17%∼19% even thereafter. From those results, it is likely that the immune system ① (TNF α → IL-12 → killer T cell system) was suppressed by the chemotherapy, the radiotherapy and the steroid administration. On the other hand, it was shown that the immune system ② (NKT cell activation → perforin) on which NKT acted was sustained.

### Industrial Applicability

In accordance with the present invention, in a cascade in which cancer immunity-bearing and activated NKT cells are participated, usefulness of a saccharide substance having an α1→3 steric structure was found to be a substance selectively acting on NKR-P1. It was found that the substance stimulates NKR-P1 of the NKT cells, enhances the production of IFN-γ, induces a Th1/Th2 balance to Th1, provides a place easily acting on cancer cells and, in addition, increases the NKT cell numbers and further the production of perforin having an inhibiting action to cancer cells enhances. Thus, the present invention has achieved a revolutionary effect in immunotherapy for cancer. In the present invention, the relation between immune activity and sugar chain structure as shown in Fig. 4 was established on the basis of the measured result data in Clinical Examples and Test Examples. It was also found in the present invention that measurement of various immunological competences is inevitable in case the therapy by anticancer agent, radioactive ray or steroid is carried out. Activation of the NKT cells, that is administration of an α1→3 saccharide substance is necessary for reservation on the basis of the above result when the immune system ② is strong and that. When the immune potency of the system ② is weak, it is necessary to administer a stronger α1→3 saccharide substance in a large dose or directly into the body such as by means of injection.

## Claims

1. A composition mainly comprising a saccharide having an α1→3 steric structure where an action of NKT cells on NKR-P1 (natural killer receptor P1) which is a natural killer (NK) cell antigen receptor of NKT cells in an activating ability of natural killer T (NKT) is used as an index and being used in a formulation where the activation can be sustained.

2. A composition where a saccharide having an α1→3 steric structure is a main ingredient, **characterized in that**, the composition selectively acts on NKR-P1 (natural killer receptor P1) which is a natural killer (NK) cell antigen receptor of NKT cells in an activating ability of natural killer T (NKT) cells and is used in a formulation where the activation can be sustained.

3. A composition mainly comprising a saccharide having an α1→3 steric structure which selectively acts on NKR-P1 (natural killer receptor P1) which is a natural killer (NK) cell antigen receptor of NKT cells whereby a large-scale production of interferon γ (IFN γ) is induced and is used in a formulation which induces the ratio of T helper 1 cell to T helper 2 cell (Th1/Th2) in a direction where an immune system on which Thl mainly acts works.

4. The composition according to any of claims 1 to 3, wherein CD 3 and CD 161 which are cell surface markers are measured whereby NKR-P1 (natural killer receptor P1) which is a natural killer (NK) cell antigen receptor of NKT cells is measured and an activating ability of the natural killer T (NKT) cells is determined.

5. The composition containing a saccharide having an α1→3 steric structure as a main ingredient according to any of claims 1 to 3, wherein it is selectively used in any of the following formulations:
1) use for a joint therapy with an anticancer chemotherapeutic agent,
2) use for a joint therapy with a radiotherapy,
3) use for a joint therapy with a steroid therapy and
4) use to patients suffering from cancer where activating ability of natural killer T (NKT) cells lowers by the action to NKR-P1 (natural killer receptor P1).

6. The composition containing a saccharide having an α1→3 steric structure as a main ingredient, where CD 3 and CD 161 which are cell surface markers are measured whereby NKR-P1 (natural killer receptor P1) which is a natural killer (NK) cell antigen receptor of NKT cells is measured and an activating ability of the natural killer T (NKT) cells is determined according to any of claims 1 to 3, wherein it is used by the way selected from any of the following formulations:
1) use for a joint therapy with an anticancer chemotherapeutic agent,
2) use for a joint therapy with a radiotherapy,
3) use for a joint therapy with a steroid therapy 'and
4) use to patients suffering from cancer where activating ability of natural killer T (NKT) cells lowers by the action to NKR-P1 (natural killer receptor P1).

7. A supplementary food for health by oral administration containing the compositions mentioned in any of claims 1 to 3.

8. A supplementary food for health by oral administration containing the compositions mentioned in any of claims 1 to 3, wherein CD 3 and CD 161 which are cell surface markers are measured whereby NKR-P1 (natural killer receptor P1) which is a natural killer (NK) cell antigen receptor of NKT cells is measured and an activating ability of the natural killer T (NKT) cells is tested.

9. A supplementary food preparation for health by oral administration containing a composition, where a saccharide having an α1→3 steric structure which is mentioned in any of claims 1 to 3 is a main ingredient, wherein it is used by the way selected from any of the following formulations:
1) use for a joint therapy with an anticancer chemotherapeutic agent,
2) use for a joint therapy with a radiotherapy,
3) use for a joint therapy with a steroid therapy and
4) use to patients suffering from cancer where activating ability of natural killer T (NKT) cells lowers by the action to NKR-P1 (natural killer receptor P1).

10. The supplementary food preparation for health by oral administration containing a composition where a saccharide having an α1→3 steric structure is a main ingredient, where CD 3 and CD 161 which are cell surface markers are measured whereby NKR-P1 (natural killer receptor P1) which is a natural killer (NK) cell antigen receptor of NKT cells is measured and an activating ability of the natural killer T (NKT) cells is determined according to any of claims 1 to 3, wherein it is selectively used in any of the following formulations:
1) use for a joint therapy with an anticancer chemotherapeutic agent,
2) use for a joint therapy with a radiotherapy,
3) use for a joint therapy with a steroid therapy and
4) use to patients suffering from cancer where activating ability of natural killer T (NKT) cells lowers by the action to NKR-P1 (natural killer receptor P1).

11. A commercial medium carrying the information mentioned in any of claims 1 to 10.

12. A commercial method utilizing the information mentioned in any of claims 1 to 10.

13. A screening method for therapeutic agent for cancer where a saccharide having an α1→3 steric structure is a main ingredient, **characterized in that**, the screening is carried out using an action of NKT cells on NKR-P1 (natural killer receptor P1), which is a natural killer (NK) cell antigen receptor of NKT cells in an activating ability of natural killer T (NKT), as an index.

14. A screening method for therapeutic agent for cancer where a saccharide having an α1→3 steric structure is a main ingredient, **characterized in that**, the screening is carried out using an action of NKT cells on NKR-P1 (natural killer receptor P1), which is a natural killer (NK) cell antigen receptor in an activation of natural killer T (NKT), as an index, wherein activation of the said NKT cell is determined by measuring the NKR-P1 (natural killer receptor P1) by the measurement of CD 3 and CD 161 which are cell surface markers.

15. A testing means for judging the usefulness of a composition where a saccharide having an α1→3 steric structure is a main ingredient, **characterized in that**, the test is carried out using an action of NKT cells on NKR-P1 (natural killer receptor P1), which is a natural killer (NK) cell antigen receptor in an activating ability of natural killer T (NKT), as an index.

16. A testing means for judging the usefulness of a composition where a saccharide having an α1→3 steric structure is a main ingredient, **characterized in that**, the test is carried out using an action of NKT cells on NKR-P1 (natural killer receptor P1), which is a natural killer (NK) cell antigen receptor of NKT cells in an activating ability of natural killer T (NKT), as an index, wherein activating ability of the said NKT cell is tested by measuring the NKR-P1 (natural killer receptor P1) by the measurement of CD 3 and CD 161 which are cell surface markers.

17. A commercial method where the testing means mentioned in claim 15 or 16 is used as a supplementary means in liaison with medical organizations.
